# EUROPEAN PATENT APPLICATION

(11) **EP 4 707 300 A1**
(43) Date of publication of application: **11.03.2026**
(21) Application number: 23936483.9
(22) Date of filing: 20.12.2023
(51) Int. Cl.: C07K 16/24, A61K 39/395, A61P 35/00, A61P 37/00

(54) **ANTI-HUMAN IL-15 ANTIBODY AND USE THEREOF**

(30) Priority: 05.05.2023 CN 202310498953
(71) Applicant: Beijing Wisdomab Biotechnology Co., Ltd, Beijing 101111 (CN)
(72) Inventor: LIU, Zhigang, Beijing 100039 (CN); ZHOU, Xiaowei, Beijing 100039 (CN); HAO, Xiaobo, Beijing 100163 (CN); LIU, Yulan, Beijing 100176 (CN); HU, Junjie, Beijing 100176 (CN); WAN, Shunan, Beijing 101111 (CN)
(74) Representative: Schnappauf, Georg
(86) International application number: PCT/CN2023/140302
(87) International publication number: WO 2024/230179

(57) **Abstract**

Disclosed are an antibody binding to human IL-15, a nucleic acid molecule encoding the antibody, a vector comprising the nucleic acid molecule, a host cell comprising the nucleic acid molecule or the vector, a method for preparing and purifying the antibody and the use of the antibody.

## Description

### CROSS REFERECE TO RELATED APPLICATION

The present application claims priority to Chinese Patent Application No. 202310498953.4, filed on May 5, 2023, which is incorporated herein by reference in its entirety.

### TECHNICAL FIELD

The present application relates generally to the field of genetic engineering and antibody medicine; in particular, to antibodies binding to human IL-15 and uses thereof.

### BACKGROUND

IL-15 gene is located on human chromosome 4q31, consisting of nine exons (1-8 and 4A) and eight introns, of which four exons (5-8) encode a mature protein. The expression of IL-15 protein is limited and is tightly regulated during transcription and translation. It is primarily expressed in monocytes/macrophages and dendritic cells ^{[1]}. IL-15 is a four-α-helix bundle cytokine, and IL-15 receptors consist of IL-15Rα, IL-2Rβ, and γc, where IL-15Rα is unique to IL-15, IL-2Rβ is a common receptor for both IL-2 and IL-15, and γc (CD132) is a common receptor for many cytokines including IL-2, IL-4, IL-7, IL-9, and IL-21. IL-15Rα binds to IL-15 to form a high-affinity stable complex, which is then presented to activated T cells or NK cells to form high-affinity immune synapses with IL-2Rβ and γc^{[2]}. Because of the shared receptor subunits (IL-2Rβ and γc), IL-2 and IL-15 trigger several similar downstream signaling pathways, including Janus kinase (JAK) signal transducer and activator of transcription (STAT), which exert the physiological functions of proliferation and inhibition of apoptosis.

Vitiligo is a chronic autoimmune disease characterized by loss of pigmentation in the skin and the appearance of white plaques of different shapes and sizes on the skin or mucous membranes, caused by loss and destruction of chromatophores-melanocytes, which affects cosmetic appearance. The global incidence rate of vitiligo is 0.5%-2.0%, with no significant difference between the sexes, and its incidence varies across geographic regions. Vitiligo is classified into segmental vitiligo, non-segmental vitiligo, and mixed vitiligo. Among them, non-segmental vitiligo accounts for about 87% of young people under 30 years of age, presenting with bilateral symmetry and a systemic distribution [3]

The J.M Richmond research group discovered that a CD8⁺ memory tissue-resident T cell, TRm, exists in skin tissue, which can remain in the tissue for at least six months after formation and increase in inflammatory environments. Biopsy of lesional skin from vitiligo patients confirmed the presence of a large number of active TRms in the lesional tissue ^{[4]}. Vitiligo frequently recurs at the same location, indicating that TRm plays an important role in disease progression. IL-15 is a cytokine essential for maintaining the long-term survival of TRm, binding to receptors on the surface of TRm cells, maintaining TRm survival, and stimulating TRm to secrete IFN-γ/granzymes/perforins, thus killing melanocytes. In addition, IFN-γ can also activate the downstream JAK-STAT pathway, allowing skin keratinocytes to secrete CXCL9 and CXCL10, recruiting more melanocyte-specific CD8⁺ T cells, and amplifying inflammation through a positive feedback loop ^{[5]}. IL-15 is a potential new target for the treatment of vitiligo.

Therefore, the development of a functional antibody against IL-15 is of great significance for the treatment of IL-15-mediated diseases.

### SUMMARY OF THE INVENTION

In a first aspect, the present application provides an antibody that binds to human IL-15, comprising a heavy chain variable region comprising amino acid sequences of HCDR1, HCDR2, and HCDR3, and a light chain variable region comprising amino acid sequences of LCDR1, LCDR2, and LCDR3, wherein
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 28, the LCDR1 has the amino acid sequence set forth in SEQ ID NO: 34, the LCDR2 has the amino acid sequence set forth in SEQ ID NO: 35, and the LCDR3 has the amino acid sequence set forth in SEQ ID NO: 36;
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 29, the LCDR1 has the amino acid sequence set forth in SEQ ID NO: 37, the LCDR2 has the amino acid sequence set forth in SEQ ID NO: 38, and the LCDR3 has the amino acid sequence set forth in SEQ ID NO: 39;
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 30, the LCDR1 has the amino acid sequence set forth in SEQ ID NO: 37, the LCDR2 has the amino acid sequence set forth in SEQ ID NO: 38, and the LCDR3 has the amino acid sequence set forth in SEQ ID NO: 39;
the HCDR1 has the amino acid sequence set forth in SEQ ID NO: 31, the HCDR2 has the amino acid sequence set forth in SEQ ID NO: 32, the HCDR3 has the amino acid sequence set forth in SEQ ID NO: 33, the LCDR1 has the amino acid sequence set forth in SEQ ID NO: 40, the LCDR2 has the amino acid sequence set forth in SEQ ID NO: 41, and the LCDR3 has the amino acid sequence set forth in SEQ ID NO: 42; or
the HCDR1 has the amino acid sequence set forth in SEQ ID NO: 31, the HCDR2 has the amino acid sequence set forth in SEQ ID NO: 45, the HCDR3 has the amino acid sequence set forth in SEQ ID NO: 46, the LCDR1 has the amino acid sequence set forth in SEQ ID NO: 47, the LCDR2 has the amino acid sequence set forth in SEQ ID NO: 48, and the LCDR3 has the amino acid sequence set forth in SEQ ID NO: 49; and
wherein the amino acid sequences of the HCDRs and the LCDRs are defined according to Kabat.

In some embodiments of the first aspect, the antibody comprises a heavy chain variable region having the amino acid sequence set forth in SEQ ID NOs: 13, 19, 21, 22 or 43.

In some embodiments of the first aspect, the antibody comprises a light chain variable region having the amino acid sequence set forth in SEQ ID NOs: 14, 20, 23 or 44.

In some embodiments of the first aspect, the antibody comprises a heavy chain variable region having the amino acid sequence set forth in SEQ ID NO: 13, and a light chain variable region having the amino acid sequence set forth in SEQ ID NO: 14;
the antibody comprises a heavy chain variable region having the amino acid sequence set forth in SEQ ID NO: 19, and a light chain variable region having the amino acid sequence set forth in SEQ ID NO: 20;
the antibody comprises a heavy chain variable region having the amino acid sequence set forth in SEQ ID NO: 21, and a light chain variable region having the amino acid sequence set forth in SEQ ID NO: 20;
the antibody comprises a heavy chain variable region having the amino acid sequence set forth in SEQ ID NO: 22, and a light chain variable region having the amino acid sequence set forth in SEQ ID NO: 23; or
the antibody comprises a heavy chain variable region having the amino acid sequence set forth in SEQ ID NO: 43, and a light chain variable region having the amino acid sequence set forth in SEQ ID NO: 44.

In a second aspect, the present application provides an antibody that binds to human IL-15, wherein the antibody comprises a heavy chain variable region having an amino acid sequence that has at least 90% identity to any one of SEQ ID NOs: 13, 19, 21, 22 and 43, and a light chain variable region having an amino acid sequence that has at least 90% identity to any one of SEQ ID NOs: 14, 20, 23 and 44.

In some embodiments of the first and second aspects, the antibody is a whole antibody, a Fab fragment, an F(ab')₂ fragment, or a single chain Fv fragment (scFv); and/or
the antibody is a monoclonal antibody; and/or
the antibody further comprises a heavy chain constant region selected from IgG1 subtype, IgG2 subtype, or IgG4 subtype; and/or
the Fc fragment comprises the amino acid F at position 234, the amino acid E at position 235, and the amino acid S at position 331; and/or
the heavy chain constant region comprises the amino acid Y at position 252, the amino acid T at position 254, and the amino acid E at position 256; and/or
the antibody further comprises a light chain constant region selected from kappa subtype or lambda subtype;
wherein amino acid positions of the antibody constant regions are determined according to EU numbering.

In some embodiments of the first and second aspects, the antibody binds to human IL-15 and/or monkey IL-15; and/or the antibody is capable of inhibiting the activity of IL-15.

In a third aspect, the present application provides a nucleic acid molecule encoding the antibody of the first or second aspect.

In a fourth aspect, the present application provides a pharmaceutical composition comprising the antibody of the first or second aspect, and a pharmaceutically acceptable excipient, diluent, or carrier.

In a fifth aspect, the present application provides use of the antibody of the first or second aspect, or the pharmaceutical composition of the fourth aspect, in the manufacture of a medicament for the prevention or treatment of an IL-15-mediated disease.

In a sixth aspect, the present application provides a method for preventing or treating an IL-15-mediated disease comprising administering to a subject in need thereof the antibody of the first or second aspect, or the pharmaceutical composition of the fourth aspect.

### BRIEF DESCRIPTION OF THE DRAWINGS

Figure 1 shows binding ability of anti-human IL-15 monoclonal antibodies to IL-15 from different species.
Figure 2 shows results of epitope analysis of the anti-human IL-15 monoclonal antibodies. Figure 2A shows results of blocking the binding of AMG714 purified phage to human IL-15 by the anti-human IL-15 monoclonal antibodies. Figure 2B shows results of blocking the binding of CALY-002 purified phage to human IL-15 by the anti-human IL-15 monoclonal antibodies.
Figure 3 shows results of the activities of the anti-human IL-15 monoclonal antibodies on HEK-blue IL-2 cells.
Figure 4 shows results of the activities of the anti-human IL-15 monoclonal antibodies on NK-92 cells.

### DESCRIPTION OF SEQUENCES

SEQ ID NO: 1 shows the amino acid sequence of the extracellular region of human *(homo sapiens)* IL-15 (hIL-15).
SEQ ID NO: 2 shows the amino acid sequence of the extracellular region of human *(homo sapiens)* IL-15Ra (hIL-15Ra).
SEQ ID NO: 3 shows the amino acid sequence of the extracellular region of cynomolgus monkey (*Macaca fascicularis*) IL-15 (mfIL-15).
SEQ ID NO: 4 shows the amino acid sequence of the extracellular region of cynomolgus monkey (*Macaca fascicularis*) IL-15Ra (mfIL-15Ra).
SEQ ID NO: 5 shows the amino acid sequence of the extracellular region of mouse *(Mus musculus)* IL-15 (mIL-15).
SEQ ID NO: 6 shows the amino acid sequence of the extracellular region of mouse *(Mus musculus)* IL-15Ra (mIL-15Ra).
SEQ ID NO: 7 shows the amino acid sequence of a His tag (His).
SEQ ID NO: 8 shows the amino acid sequence of the Fc fragment of mouse IgG1 antibody (mFc1).
SEQ ID NO: 9 shows the amino acid sequence of the heavy chain constant region of human *(homo sapiens)* IgG1 subtype.
SEQ ID NO: 10 shows the amino acid sequence of the heavy chain constant region of human IgGlm3 subtype.
SEQ ID NO: 11 shows the amino acid sequence of the light chain constant region of human *(homo sapiens)* kappa subtype.
SEQ ID NO: 12 shows the amino acid sequence of the light chain constant region of human *(homo sapiens)* lambda subtype.
SEQ ID NO: 13 shows the amino acid sequence of heavy chain variable region R1A3VH of anti-human IL-15 monoclonal antibody R1A3.
SEQ ID NO: 14 shows the amino acid sequence of light chain variable region R1A3VK of anti-human IL-15 monoclonal antibody R1A3.
SEQ ID NO: 15 shows the amino acid sequence of the heavy chain variable region of anti-human IL-15 monoclonal antibody AMG714.
SEQ ID NO: 16 shows the amino acid sequence of the light chain variable region of anti-human IL-15 monoclonal antibody AMG714.
SEQ ID NO: 17 shows the amino acid sequence of the heavy chain variable region of anti-human IL-15 monoclonal antibody CALY-002.
SEQ ID NO: 18 shows the amino acid sequence of the light chain variable region of anti-human IL-15 monoclonal antibody CALY-002.
SEQ ID NO: 19 shows the amino acid sequence of heavy chain variable region R4G4VH of anti-human IL-15 monoclonal antibody R4G4VH+R22F11VK.
SEQ ID NO: 20 shows the amino acid sequence of light chain variable region R22F11VK of anti-human IL-15 monoclonal antibodies R4G4VH+R22F11VK and R2H2VH+R22F11VK.
SEQ ID NO: 21 shows the amino acid sequence of heavy chain variable region R2H2VH of anti-human IL-15 monoclonal antibody R2H2VH+R22F11VK.
SEQ ID NO: 22 shows the amino acid sequence of heavy chain variable region R26H10VH of anti-human IL-15 monoclonal antibody R26H10.
SEQ ID NO: 23 shows the amino acid sequence of light chain variable region R26H10VK of anti-human IL-15 monoclonal antibody R26H10.
SEQ ID NO: 24 shows the nucleotide sequence of primer PmCGR.
SEQ ID NO: 25 shows the nucleotide sequence of primer PmCKR.
SEQ ID NO: 26 shows the amino acid sequence of HCDR1 of heavy chain variable regions R1A3VH, R4G4VH and R2H2VH.
SEQ ID NO: 27 shows the amino acid sequence of HCDR2 of heavy chain variable regions R1A3VH, R4G4VH and R2H2VH.
SEQ ID NO: 28 shows the amino acid sequence of HCDR3 of heavy chain variable region R1A3VH.
SEQ ID NO: 29 shows the amino acid sequence of HCDR3 of heavy chain variable region R4G4VH.
SEQ ID NO: 30 shows the amino acid sequence of HCDR3 of heavy chain variable region R2H2VH.
SEQ ID NO: 31 shows the amino acid sequence of HCDR1 of heavy chain variable regions R26H10VH and R6E11VH.
SEQ ID NOs: 32-33 show the amino acid sequences of HCDR2 and HCDR3 of heavy chain variable region R26H10VH, respectively.
SEQ ID NOs: 34-36 show the amino acid sequences of LCDR1, LCDR2, and LCDR3 of light chain variable region R1A3VK, respectively.
SEQ ID NOs: 37-39 show the amino acid sequences of LCDR1, LCDR2, and LCDR3 of light chain variable region R22F11VK, respectively.
SEQ ID NOs: 40-42 show the amino acid sequences of LCDR1, LCDR2, and LCDR3 of light chain variable region R26H10VK, respectively.
SEQ ID NO: 43 shows the amino acid sequence of the heavy chain variable region R6E11VH of anti-human IL-15 monoclonal antibody R6E11.
SEQ ID NO: 44 shows the amino acid sequence of the light chain variable region R6E11VK of anti-human IL-15 monoclonal antibody R6E11.
SEQ ID NOs: 45-46 show the amino acid sequences of HCDR2 and HCDR3 of heavy chain variable region R6E11VH, respectively.
SEQ ID NOs: 47-49 show the amino acid sequences of LCDR1, LCDR2, and LCDR3 of light chain variable region R6E11VK, respectively.
SEQ ID NO: 50 shows the amino acid sequence of the heavy chain variable region of DP47 antibody.
SEQ ID NO: 51 shows the amino acid sequence of the light chain variable region of DP47 antibody.

### DETAILED DESCRIPTION OF THE INVENTION

The inventors of the present application have obtained novel anti-human IL-15 antibodies by antibody engineering techniques. In various aspects of the present application, provided are novel anti-human IL-15 antibodies, nucleic acid molecules encoding the antibodies, vectors comprising the nucleic acid molecules, host cells comprising the nucleic acid molecules or vectors, methods for preparing and purifying the antibodies, and medical and biological applications of the antibodies. A full-length antibody molecule can be constructed from the amino acid sequences of the variable regions of the antibodies provided herein and used as a medicament for preventing or treating human IL-15-mediated diseases.

Unless otherwise indicated, the inventions of the present application can be implemented using conventional molecular biology, microbiology, cell biology, biochemistry, and immunological techniques in the art.

Unless otherwise indicated, the terms used in the present application have the meanings commonly understood by those skilled in the art.

### DEFINITIONS

As used herein, the term "antibody" refers to an immunoglobulin molecule that is capable of specifically binding to a target via at least one antigen recognition site located in a variable region of the immunoglobulin molecule. Targets include, but are not limited to, carbohydrates, polynucleotides, lipids, polypeptides, and the like. As used herein, an "antibody" includes not only an intact *(i.e.,* full-length) antibody, but also an antigen-binding fragment thereof (for example, Fab, Fab', F(ab')₂, or Fv), a variant thereof, a fusion protein comprising portions of an antibody, a humanized antibody, a chimeric antibody, a diabody, a linear antibody, a single chain antibody, a multi-specific antibody (for example, a bispecific antibody), and any other modified configurations of an immunoglobulin molecule comprising a desired specific antigen recognition site, including a glycosylated variant of an antibody, an amino acid sequence variant of an antibody, and a covalently modified antibody.

Typically, an intact or full-length antibody comprises two heavy chains and two light chains. Each heavy chain contains a heavy chain variable region (VH) and first, second, and third constant regions (CH1, CH2 and CH3). Each light chain contains a light chain variable region (VL) and a constant region (CL). A full-length antibody may be of any type of antibody, such as an IgD, IgE, IgG, IgA, or IgM (or their subtypes) antibody, but it is not necessary for the antibody to belong to any particular type. Immunoglobulins can be assigned to different types depending on their amino acid sequences of the heavy chain constant domains. Generally, immunoglobulins have five main types: IgA, IgD, IgE, IgG, and IgM, and some of these types can be further classified into subtypes (isotypes), such as IgG1, IgG2, IgG3, IgG4, IgA1, and IgA2. Heavy chain constant domains corresponding to individual immunoglobulin types are referred to as α, δ, ε, γ, and µ, respectively. Subunit structures and three-dimensional structures of different types of immunoglobulins are well known.

As used herein, the term "antigen-binding fragment or antigen-binding portion" refers to a portion or region of an intact antibody molecule that is responsible for binding to an antigen. The antigen-binding domain may comprise a heavy chain variable region (VH), a light chain variable region (VL), or both. Each of VH and VL typically contains three complementarity determining regions, CDR1, CDR2, and CDR3.

It is well known to those skilled in the art that complementarity determining regions (CDRs, usually including CDR1, CDR2 and CDR3) are the regions of a variable region that have the greatest impact on the affinity and specificity of an antibody. The CDR amino acid sequences in VH or VL have two common definitions, i.e., the Chothia definition and the kabat definition. See, e.g., Kabat, "Sequences of Proteins of Immunological Interest", National Institutes of Health, Bethesda, Md. (1991) ⁷; A1-Lazikani et al., J. Mol. Biol. 273:927-948 (1997) ⁸; and Martin et al., Proc. Natl. Acad. Sci. USA 86:9268-9272 (1989) ⁹. For the variable region amino acid sequences of a given antibody, the CDR amino acid sequences in the VH and VL amino acid sequences can be determined according to the Chothia definition or the Kabat definition. In an embodiment of the present application, the CDR amino acid sequences are defined according to Kabat.

For the variable region amino acid sequences of a given antibody, the CDR amino acid sequences in the variable region amino acid sequences can be determined in a variety of ways, for example, using online software Abysis (http://www.abysis.org/).

Examples of an antigen-binding fragment include, but are not limited to: (1) a Fab fragment, which may be a monovalent fragment having a VL-CL chain and a VH-CH1 chain; (2) an F(ab')₂ fragment, which may be a divalent fragment having two Fab' fragments linked by a disulfide bridge of the hinge region *(i.e.,* a dimer of Fab'); (3) an Fv fragment having VL and VH domains in a single arm of an antibody; (4) a single chain Fv (scFv), which may be a single polypeptide chain consisting of a VH domain and a VL domain via a polypeptide linker; and (5) (scFv)₂, which may comprise two VH domains linked by a peptide linker and two VL domains that are combined with the two VH domains via a disulfide bridge.

As used herein, the term "specific binding" refers to a non-random binding reaction between two molecules, e.g., binding of an antibody to an antigen epitope.

As used herein, the term "monoclonal antibody" refers to an antibody from a population of substantially homogeneous antibodies, which means that the antibodies constituting the population are the same except for naturally occurring mutations which may occur in a small number of individual antibodies.

As used herein, the term "humanized antibody" refers to a constant region portion (i.e., CH and CL regions) of an antibody or an antibody that is all encoded by human antibody genes. The humanized antibody can greatly reduce the immune side effects caused by heterologous antibodies in the human body. Humanized antibodies include chimeric antibodies, modified antibodies, and fully humanized antibodies, and the like.

In a first aspect, the present application provides an antibody that binds to human IL-15, comprising a heavy chain variable region comprising amino acid sequences of HCDR1, HCDR2, and HCDR3, and a light chain variable region comprising amino acid sequences of LCDR1, LCDR2, and LCDR3, wherein
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 28, the LCDR1 has the amino acid sequence set forth in SEQ ID NO: 34, the LCDR2 has the amino acid sequence set forth in SEQ ID NO: 35, and the LCDR3 has the amino acid sequence set forth in SEQ ID NO: 36;
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 29, the LCDR1 has the amino acid sequence set forth in SEQ ID NO: 37, the LCDR2 has the amino acid sequence set forth in SEQ ID NO: 38, and the LCDR3 has the amino acid sequence set forth in SEQ ID NO: 39;
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 30, the LCDR1 has the amino acid sequence set forth in SEQ ID NO: 37, the LCDR2 has the amino acid sequence set forth in SEQ ID NO: 38, and the LCDR3 has the amino acid sequence set forth in SEQ ID NO: 39;
the HCDR1 has the amino acid sequence set forth in SEQ ID NO: 31, the HCDR2 has the amino acid sequence set forth in SEQ ID NO: 32, the HCDR3 has the amino acid sequence set forth in SEQ ID NO: 33, the LCDR1 has the amino acid sequence set forth in SEQ ID NO: 40, the LCDR2 has the amino acid sequence set forth in SEQ ID NO: 41, and the LCDR3 has the amino acid sequence set forth in SEQ ID NO: 42; or
the HCDR1 has the amino acid sequence set forth in SEQ ID NO: 31, the HCDR2 has the amino acid sequence set forth in SEQ ID NO: 45, the HCDR3 has the amino acid sequence set forth in SEQ ID NO: 46, the LCDR1 has the amino acid sequence set forth in SEQ ID NO: 47, the LCDR2 has the amino acid sequence set forth in SEQ ID NO: 48, and the LCDR3 has the amino acid sequence set forth in SEQ ID NO: 49;
wherein the amino acid sequences of the HCDRs and the LCDRs are defined according to Kabat.

In some embodiments of the first aspect, the antibody comprises a heavy chain variable region having the amino acid sequence set forth in SEQ ID NO: 13.

In some embodiments of the first aspect, the antibody comprises a heavy chain variable region having the amino acid sequence set forth in SEQ ID NO: 19.

In some embodiments of the first aspect, the antibody comprises a heavy chain variable region having the amino acid sequence set forth in SEQ ID NO: 21.

In some embodiments of the first aspect, the antibody comprises a heavy chain variable region having the amino acid sequence set forth in SEQ ID NO: 22.

In some embodiments of the first aspect, the antibody comprises a heavy chain variable region having the amino acid sequence set forth in SEQ ID NO: 43.

In some embodiments of the first aspect, the antibody comprises a light chain variable region having the amino acid sequence set forth in SEQ ID NO: 14.

In some embodiments of the first aspect, the antibody comprises a light chain variable region having the amino acid sequence set forth in SEQ ID NO: 20.

In some embodiments of the first aspect, the antibody comprises a light chain variable region having the amino acid sequence set forth in SEQ ID NO: 23.

In some embodiments of the first aspect, the antibody comprises a light chain variable region having the amino acid sequence set forth in SEQ ID NO: 44.

In some embodiments of the first aspect, the antibody comprises a heavy chain variable region having the amino acid sequence set forth in SEQ ID NO: 13, and a light chain variable region having the amino acid sequence set forth in SEQ ID NO: 14.

In some embodiments of the first aspect, the antibody comprises a heavy chain variable region having the amino acid sequence set forth in SEQ ID NO: 19, and a light chain variable region having the amino acid sequence set forth in SEQ ID NO: 20.

In some embodiments of the first aspect, the antibody comprises a heavy chain variable region having the amino acid sequence set forth in SEQ ID NO: 21, and a light chain variable region having the amino acid sequence set forth in SEQ ID NO: 20.

In some embodiments of the first aspect, the antibody comprises a heavy chain variable region having the amino acid sequence set forth in SEQ ID NO: 22, and a light chain variable region having the amino acid sequence set forth in SEQ ID NO: 23.

In some embodiments of the first aspect, the antibody comprises a heavy chain variable region having the amino acid sequence set forth in SEQ ID NO: 43, and a light chain variable region having the amino acid sequence set forth in SEQ ID NO: 44.

In a second aspect, the present application provides an antibody that binds to human IL-15, wherein the antibody comprises a heavy chain variable region having an amino acid sequence that has at least 90% identity to any one of SEQ ID NOs: 13, 19, 21, 22 and 43, and a light chain variable region having an amino acid sequence that has at least 90% identity to any one of SEQ ID NOs: 14, 20, 23 and 44.

In some embodiments of the second aspect, the amino acid sequence of the heavy chain variable region of the antibody has at least 90%, 91%, 92%, 93%, 94%, 95%, 96%, 97%, 98%, 99% or higher identity to any one of SEQ ID NOs: 13, 19, 21, 22 and 43.

In some embodiments of the second aspect, the amino acid sequence of the light chain variable region of the antibody has at least 90%, 91%, 92%, 93%, 94%, 95%, 96%, 97%, 98%, 99% or higher identity to any one of SEQ ID NOs: 14, 20, 23 and 44.

In some embodiments of the second aspect, the amino acid sequence of the heavy chain variable region of the antibody differs from the amino acid sequence set forth in any one of SEQ ID NOs: 13, 19, 21, 22 and 43 by about 1, 2, 3, 4, 5, 6, 7, 8, 9 or 10 amino acid substitutions, deletions, and/or additions.

In some embodiments of the second aspect, the amino acid sequence of the light chain variable region of the antibody differs from the amino acid sequence set forth in any one of SEQ ID NOs: 14, 20, 23 and 44 by about 1, 2, 3, 4, 5, 6, 7, 8, 9 or 10 amino acid substitutions, deletions, and/or additions.

In some embodiments of the second aspect, the C-terminal or N-terminal region of the amino acid sequence set forth in any one of SEQ ID NOs: 13, 19, 21, 22 and 43 may also be truncated by about 1, 2, 3, 4, 5, 6, 7, 8, 9, 10, 11, 12, 13, 14, 15, 20, 25 or more amino acids, while still retaining a function similar to that of the heavy chain variable region of the antibody.

In some embodiments of the second aspect, 1, 2, 3, 4, 5, 6, 7, 8, 9, 10, 11, 12, 13, 14, 15, 20, 25 or more amino acids may also be added to the C-terminal or N-terminal region of the amino acid sequence set forth in any one of SEQ ID NOs: 13, 19, 21, 22 and 43, and the resulting amino acid sequence still retains a function similar to that of the heavy chain variable region of the antibody.

In some embodiments of the second aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in any one of SEQ ID NOs: 13, 19, 21, 22 and 43, provided that the altered amino acid sequence substantially retains a function similar to that of the heavy chain variable region of the antibody.

In some embodiments of the second aspect, the C-terminal or N-terminal region of the amino acid sequence set forth in any one of SEQ ID NOs: 14, 20, 23 and 44 may also be truncated by about 1, 2, 3, 4, 5, 6, 7, 8, 9, 10, 11, 12, 13, 14, 15, 20, 25 or more amino acids, while still retaining a function similar to that of the light chain variable region of the antibody.

In some embodiments of the second aspect, 1, 2, 3, 4, 5, 6, 7, 8, 9, 10, 11, 12, 13, 14, 15, 20, 25 or more amino acids may also be added to the C-terminal or N-terminal region of the amino acid sequences set forth in any one of SEQ ID NOs: 14, 20, 23 and 44, the resulting amino acid sequence still retains a function similar to that of the light chain variable region of the antibody.

In some embodiments of the second aspect, 1, 2, 3, 4, 5, 6, 7, 8, 9, 10, 11, 12, 13, 14, 15, 20, 25 or more amino acids may also be added or deleted at regions other than the C-terminus or the N-terminus of the amino acid sequence set forth in any one of SEQ ID NOs: 14, 20, 23 and 44, provided that the altered amino acid sequence substantially retains a function similar to that of the light chain variable region of the antibody.

In some embodiments of the first and second aspects, the antibody is a whole antibody, a Fab fragment, an F(ab')₂ fragment, or a single chain Fv fragment (scFv).

In some embodiments of the first and second aspects, the antibody is a fully human antibody or a humanized antibody.

In some embodiments of the first and second aspects, the antibody is a monoclonal antibody.

In some embodiments of the first and second aspects, the antibody further comprises a heavy chain constant region selected from IgG1 subtype, IgG2 subtype, or IgG4 subtype.

In some particular embodiments of the first and second aspects, the heavy chain constant region is of IgG1 subtype, e.g., IgG1m3 subtype.

In some embodiments of the first aspect and the second aspect, the Fc fragment comprises the amino acid F at position 234, the amino acid E at position 235, and the amino acid S at position 331; wherein amino acid positions of the antibody constant region are determined according to EU numbering.

In some embodiments of the first aspect and the second aspect, the heavy chain constant region comprises the amino acid Y at position 252, the amino acid T at position 254, and the amino acid E at position 256; wherein amino acid positions of the antibody constant region are determined according to EU numbering.

In some embodiments of the first aspect and the second aspect, the antibody further comprises a light chain constant region selected from kappa subtype or lambda subtype.

In some particular embodiments of the first aspect and the second aspect, the antibody further comprises a light chain constant region selected from kappa subtype.

In some embodiments of the first and second aspects, the antibody binds to human IL-15.

In some particular embodiments of the first and second aspects, the antibody binds to recombinant human IL-15 (SEQ ID NO: 1).

In some embodiments of the first and second aspects, the antibody binds to monkey IL-15.

In some particular embodiments of the first and second aspects, the antibody binds to recombinant monkey IL-15 (SEQ ID NO: 3).

In some embodiments of the first and second aspects, the antibody binds to a complex of IL-15 and IL-15α, such as a complex of human IL-15 and human IL-15α, or a complex of monkey IL-15 and monkey IL-15α.

In some embodiments of the first and second aspects, the antibody is capable of inhibiting the activity of IL-15.

In some particular embodiments of the first and second aspects, the antibody inhibits the ability of IL-15 to induce secretion of secreted embryonic alkaline phosphatase (SEAP).

In some particular embodiments of the first and second aspects, the antibody inhibits the ability of IL-15 to induce proliferation of NK92 cells.

In some embodiments of the first and second aspects, the antibody does not bind to murine IL-15, e.g., recombinant mouse IL-15 (SEQ ID NO: 5).

In a third aspect, the present application provides a nucleic acid molecule encoding the antibody of the first or second aspect.

In some embodiments of the third aspect, the nucleic acid molecule may include a DNA molecule and an RNA molecule. The nucleic acid molecule may be single-stranded or doublestranded and may be cDNA.

In some embodiments of the third aspect, the nucleic acid molecule is operably linked to a regulatory amino acid sequence that can be recognized by a host cell transformed with the vector.

In a fourth aspect, the present application provides a pharmaceutical composition comprising the antibody of the first or second aspect and a pharmaceutically acceptable excipient, diluent, or carrier.

In some embodiments of the fourth aspect, the pharmaceutical composition is used to prevent or treat an IL-15-mediated disease.

In some embodiments of the fourth aspect, the IL-15-mediated disease is selected from the group consisting of vitiligo, celiac disease, eosinophilic esophagitis, and large granular lymphocytic leukemia.

In some embodiments of the fourth aspect, the pharmaceutical composition may further comprise one or more of the following: a lubricant, such as talc, magnesium stearate, and mineral oil; a wetting agent; an emulsifier; a suspending agent; a preservative such as benzoic acid, sorbic acid and calcium propionate; a sweetener and/or a flavoring agent, and the like.

In some embodiments of the fourth aspect, the pharmaceutical composition herein may be formulated as a tablet, a pill, a powder, a lozenge, an elixir, a suspension, an emulsion, a solution, a syrup, a suppository, a capsule, and the like.

In some embodiments of the fourth aspect, the pharmaceutical composition of the present application may be delivered using any physiologically acceptable administration route which includes, but is not limited to, oral administration, parenteral administration, nasal administration, rectal administration, intraperitoneal administration, intravascular injection, subcutaneous administration, transdermal administration, inhalation administration, or the like.

In some embodiments of the fourth aspect, the pharmaceutical composition for therapeutic uses may be formulated for storage in the form of a lyophilized formulation or an aqueous solution by mixing a reagent of desired purity with a pharmaceutically acceptable carrier or excipient, as appropriate.

In a fifth aspect, the present application provides use of the antibody of the first or second aspect, and the pharmaceutical composition of the fourth aspect, in the manufacture of a medicament for the prevention or treatment of an IL-15-mediated disease.

In some embodiments of the fifth aspect, the IL-15-mediated disease is selected from the group consisting of vitiligo, celiac disease, eosinophilic esophagitis, and large granular lymphocytic leukemia.

In a sixth aspect, the application provides a method for preventing or treating an IL-15-mediated disease comprising administering to a subject in need thereof the antibody of the first aspect or the antibody of the second aspect, or the pharmaceutical composition of the fourth aspect.

In some embodiments of the sixth aspect, the IL-15-mediated disease is selected from the group consisting of vitiligo, celiac disease, eosinophilic esophagitis, and large granular lymphocytic leukemia.

In other aspects, the present application also provides a vector comprising the nucleic acid molecule, a host cell comprising the nucleic acid molecule or the vector, and a method for producing the antibody. In some embodiments, the method for producing the antibody comprises culturing the host cell to facilitate expression of the nucleic acid. In some embodiments, the method for producing the antibody further comprises recovering the antibody from the host cell culture medium.

It is to be understood that the foregoing detailed description is intended only to enable those skilled in the art to have a better understanding of the present application and is not intended to cause limitations in any way. Various modifications and variations can be made to the described embodiments by those skilled in the art.

### Examples

The following Examples are for purposes of illustration only and are not intended to limit the scope of the present application.

### Example 1: Preparation of recombinant proteins

In the preparation and characterization of the IL-15 specific antibodies, a variety of recombinant proteins were utilized, including a superagonist composed of co-expressed human IL-15 (hIL15, SEQ ID NO: 1) and human IL-15Rα (hIL-15Rα, SEQ ID NO: 2), a superagonist composed of co-expressed monkey IL-15 (mfIL-15, SEQ ID NO: 3) and monkey IL-15Rα (mfIL-15Rα, SEQ ID NO: 4), and a superagonist composed of co-expressed mouse IL-15 (mIL-15, SEQ ID NO: 5) and mouse IL-15Rα (mIL-15Rα, SEQ ID NO: 6). Addition of a His tag (His, SEQ ID NO: 7) or a Fc fragment (mFc1, SEQ ID NO: 8) of the mouse IgG1 antibody to the C-terminus of the IL-15Rα, facilitated purification and functional identification of the recombinant protein. In the preparation of a recombinant antibody, the heavy chain constant region of the antibody may be a human IgG1 subtype (SEQ ID NO: 9) or various mutants of a selected human IgG1 subtype, e.g., IgG1m3 (SEQ ID NO: 10); and the light chain constant region may be a human kappa subtype (SEQ ID NO: 11) or a human lambda subtype (SEQ ID NO: 12).

Genes (including His or mFc1 tags) for the various recombinant proteins described above were designed and synthesized according to the amino acid sequences of the recombinant proteins in the Uniprot database. The synthesized genes for the various recombinant proteins were cloned into a suitable eukaryotic expression vector (such as pcDNA3.1 from Invitrogen Inc., and the like) using conventional techniques in molecular biology. Plasmids prepared for recombinant protein expression were then transfected with liposomes (such as 293fectin from Invitrogen Inc., and the like) or another cationic transfection reagent (such as PEI, and the like) into HEK293 cells (such as HEK293F from Invitrogen Inc., and the like), which were cultured in suspension under a serum-free condition for 3 to 4 days. The culture supernatant was then harvested by centrifugation or the like.

Recombinant proteins expressed as fusions with His tags were subjected to one-step purification from the culture supernatant using a metal chelate affinity chromatography column (such as HisTrap FF from GE Inc., and the like). Antibodies and recombinant proteins expressed as fusions with Fc tags were subjected to one-step purification using a ProteinA/G affinity chromatography column (such as Mabselect SURE from GE Inc., and the like). The preservation buffer for the recombinant proteins was then replaced with PBS (pH 7.0) or another suitable buffer using a desalting column (such as Hitrap desaulting from GE Inc., and the like). After sterilization by filtration, the proteins were stored in aliquots at -20°C for later use.

### Example 2: Screening of anti-hIL-15 monoclonal antibodies from fully human Fab phage library

### 2.1 Screening of fully human Fab library

A prepared fully human Fab phage library (see Chinese Patent Application No. 202210871809.6) ^{[7]} was screened by a solid phase screening strategy (as for the experimental protocol, see Phage Display: A Practical Approach, Clackson, T. (USA) and Lowman, H. B. (USA) (ed.), translated by Lan Ma et al., Chemical Industry Press, May 2008) ^{[6]} using the recombinant human IL-15+IL-15Rα superagonist prepared in Example 1 as an antigen. Three rounds of screening were carried out by means of binding, elution, neutralization, infection, and amplification. Finally, one monoclonal antibody, R1A3 (the amino acid sequence of its heavy chain variable region R1A3VH is set forth in SEQ ID NO: 13; and the amino acid sequence of its light chain variable region R1A3VK is set forth in SEQ ID NO: 14), which specifically binded to human IL-15, was obtained.

R1A3 was prepared as a whole IgG1m3 subtype antibody using conventional means in molecular biology. In addition, the heavy chain variable region (SEQ ID NO: 15) and light chain variable region (SEQ ID NO: 16) of AMG714 were synthesized with reference to U.S. Patent No. US7153507B2 ^{[8]}, and AMG714 was prepared as a whole IgG1m3 subtype antibody for control studies. Similarly, the heavy chain variable region (SEQ ID NO: 17) and the light chain variable region (SEQ ID NO: 18) of CALY-002 were synthesized with reference to U.S. Patent No. US10301384B2 ^{[9]}, and CALY-002 was prepared as a whole IgG1m3 subtype antibody for control studies.

### 2.2 Affinity analysis of anti-human IL-15 monoclonal antibody R1A3

The affinity of the anti-IL-15 monoclonal antibody was determined by the surface plasmon resonance technique using Biacore T200. Related reagents and consumables such as the Amine Coupling Kit (BR-1000-50), the Human Antibody Capture Kit (BR-1008-39), a Series S CM5 chip (14100530), and 10×HBS-EP (BR100669) at pH 7.4 were purchased from GE healthcare. The surface of the carboxylated CM5 chip was activated with 1-Ethyl-3-(3-dimethylaminopropyl) carbodiimide hydrochlorid (EDC) and N-Hydroxysuccinimide (NHS) according to the manual in the kit. An anti-human IgG (Fc) antibody (capture antibody) was diluted to 25 µg/mL with 10mM sodium acetate (pH 5.0), and then injected at a flow rate of 10µL/min to achieve a coupling amount of approximately 10,000 response units (RU). Followed by the injection of capture antibody, 1M ethanolamine was injected to block unreacted groups. For the kinetic measurement, the anti-IL-15 monoclonal antibody was diluted to 1µg/mL, and injected at 10µL/min to ensure that approximately 200RU of the antibody was captured by the anti-human Fc antibody. IL-15+IL-15Rα complex was then set to a serial concentration gradient (such as 1.23nM, 3.7nM, 11.1nM, 33.3nM, and 100nM), and injected at a flow rate of 30µL/min from lower to higher concentrations, with a binding time of 90s and a dissociation time of 1,200s. 3M MgCl₂ was injected at a flow rate of 10µL/min for a total of 30s to regenerate the surface of the chip. The association rate (Kₐ) and dissociation rate (K_{d}) were calculated by fitting the binding and dissociation sensorgrams with a 1:1 binding model using Biacore T200 evaluation software version 3.2.1. The dissociation equilibrium constant (K_{D}) was calculated as the ratio K_{d}/Kₐ. The fitting results are shown in Table 1.

**Table 1. Affinity constants for binding of anti-human IL-15 monoclonal antibody to human IL-15 (IL-15+IL-15Rα complex)**

| | Kₐ(M⁻¹s⁻¹ ) | K_{d}(s⁻¹) | K_{D} (M) |
|---|---|---|---|
| AMG714 | 1.679E+6 | 1.219E-3 | 7.259E-10 |
| CALY-002 | 1.337E+6 | 3.190E-4 | 2.386E-10 |
| R1A3 | 1.410E+6 | 5.371E-4 | 3.810E-10 |

### Example 3: Preparation of R1A3 mutants

### 3.1 Preparation of library of R1A3 recombinant mutants

A library of CDR3 mutants based on R1A3VH was constructed by introducing mutations into CDR3 of heavy chain variable region R1A3VH using conventional means in molecular biology. The mutation scheme was designed as shown in Table 2, with a constructed library capacity of 2E+7 and an accuracy of 82%.

**Table 2. Mutation scheme of library of CDR3 mutants based on R1A3VH**

| Original amino acid | Designed amino acid | Degenerate codon |
|---|---|---|
| G | G, V, A, or D | GNT |
| G | G, V, A, or D | GNT |
| H | H, Q, N, K, D, or E | VAM |
| W | W | TGG |
| N | H, Q, N, K, D, or E | VAM |
| A | C, Y, S, A, D, or G | KVT |
| F | F, S, Y, I, N, orT | WHT |
| D | D, E, N, K, H, or Q | VAM |
| F | F, S, Y, I, N, or T | WHT |

### 3.2 Screening of library of R1A3 recombinant mutants

Based on the recombinant library phage display system (see Example 1 in Chinese Patent Application No. 202210871809.6), the prepared library of R1A3 heavy chain mutants was recombined with the library of fully human light chain antibodies to construct a library of recombinant Fab mutants. The prepared library of recombinant Fab mutants was screened by a solid phase screening strategy (as for the experimental protocol, see Phage Display: A Practical Approach, Clackson, T. (USA) and Lowman, H. B. (USA) (ed.), translated by Lan Ma et al., Chemical Industry Press, May 2008) using the recombinant human IL-15+IL-15Rα superagonist prepared in Example 1 as an antigen. Three rounds of screening were carried out by means of binding, elution, neutralization, infection, and amplification. Finally, the anti-human IL-15 monoclonal antibodies, R4G4VH+R22F11VK (the amino acid sequence of its heavy chain variable region R4G4VH is set forth in SEQ ID NO: 19; and the amino acid sequence of its light chain variable region R22F11VK is set forth in SEQ ID NO: 20) and R2H2VH+R22F11VK (the amino acid sequence of its heavy chain variable region R2H2VH is set forth in SEQ ID NO: 21; and the amino acid sequence of its light chain variable region R22F11VK is set forth in SEQ ID NO: 20), were obtained.

### 3.3 Affinity assay of R1A3 mutants

Referring to Example 2.2, the affinity of the anti-human IL-15 monoclonal antibody R1A3 and its mutants was analyzed with Biacore T200. Results are shown in Table 3.

**Table 3. Affinity constants for binding of anti-human IL-15 monoclonal antibody R1A3 and its mutants to human IL-15 (IL-15+IL-15Rα complex)**

| | Kₐ (M⁻¹s⁻¹) | K_{d} (s⁻¹) | K_{D} (M) |
|---|---|---|---|
| R4G4VH+R22F11VK | 8.377E+5 | 2.898E-4 | 3.459E-10 |
| R2H2VH+R22F11VK | 7.910E+5 | 2.994E-4 | 3.786E-10 |
| R1A3 | 1.555E+6 | 5.481E-4 | 3.525E-10 |
| AMG714 | 1.775E+6 | 1.110E-3 | 6.249E-10 |

### Example 4: Screening of mouse anti-human IL-15 monoclonal antibodies from mouse immune library

### 4.1 Preparation of hIL-15 mouse immune library

6-8 week-old BALB/c mice were immunized with recombinant proteins hIL-15+hIL-15Rα and mfIL-15+mfIL-15Rα superagonists prepared in Example 1 as antigens. The immunization dose was 50µg per mouse, with booster immunizations every 14 days. Mice were euthanized 8 weeks after the priming, and collected for splenocytes. Mouse splenic lymphocytes were isolated with the Mouse Lymphocyte Separation Medium (Dakewe Biotech Co., Ltd., CAT#DKW33-R0100). Total RNA was extracted from the isolated lymphocytes with a cellular total RNA extraction kit (TIANGEN Biotech (BEIJING) Co., Ltd., CAT#DP430). The extracted total RNA was used as a template to synthesize the heavy chain variable regions and light chain variable regions of antibodies with the First Strand cDNA Synthesis Kit (Thermo scientific, CAT#K1621), respectively. Gene-specific primers were used as primers for reverse transcription. The primers annealed to regions located in the heavy chain constant regions and light chain constant regions of antibodies, respectively, specific sequences of which were PmCGR: TGCATTTGAACTCCTTGCC (SEQ ID NO:24) and PmCKR: CCATCAATCTTCCACTTGAC (SEQ ID NO:25), respectively. The synthesized cDNAs were immediately stored at -70°C for later use. Then, the cDNAs obtained by the reverse transcription were used as templates to amplify the nucleotide sequences encoding VH and VK of the mouse antibodies by PCR with the primers synthesized according to the reference (Krebber A, Bornhauser S, Burmester J, et al., Reliable cloning of functional antibody variable domains from hybridomas and spleen cell repertoires employing a reengineered phage display system. J Immunol Methods. 1997;201(1):35-55 ^{[10]}, which is incorporated herein by reference in its entirety), respectively. The nucleotide sequences encoding VH and VK of the mouse antibodies were then cloned into recombinant vectors, pHGDisn-attP-new and pHKb-attB-new (see Example 1 in Chinese Patent Application No. 202210871809.6 for the preparation of the recombinant plasmids), respectively.

The prepared PHGDisn-attP-new-mVHs heavy chain library (with a capacity of 6.1E+7 and an accuracy of 63%) and the PHKb-attB-new-mVKs light chain library (with a capacity of 7.4E+7 and an accuracy of 78%) were recombinanted (see Example 1 of Chinese Patent Application No. 202210871809.6) to prepare a mouse recombinant phage library, which had a capacity of 2.7E+11 and a sequencing accuracy of 60%.

### 4.2 Screening of hIL-15 mouse immune library

The prepared mouse recombinant phage library was screened by a solid phase screening strategy (as for the experimental protocol, see Phage Display: A Practical Approach, Clackson, T. (USA) and Lowman, H. B. (USA) (ed.), translated by Lan Ma et al., Chemical Industry Press, May 2008) using recombinant human IL-15+IL-15Rα and monkey IL-15+IL-15Rα superagonists prepared in Example 1 as antigens. Three rounds of screening were carried out by means of binding, elution, neutralization, infection, and amplification. Finally, one mouse monoclonal antibody, R6E11 (the amino acid sequence of its heavy chain variable region R6E11VH is set forth in SEQ ID NO: 43; and the amino acid sequence of its light chain variable region R6E11VK is set forth in SEQ ID NO: 44), which specifically binded to human IL-15, was obtained.

### 4.3 Affinity analysis of mouse anti-human IL-15 monoclonal antibody R6E11

Referring to Example 2.2, the affinity of the anti-human IL-15 monoclonal antibody R6E11 was analyzed with Biacore T200. Results are shown in Table 4.

**Table 4. Affinity constants for binding of mouse anti-human IL-15 antibody R6E11 to human IL-15 (IL-15+IL-15Rα complex)**

| | Kₐ (M⁻¹s⁻¹ ) | K_{d} (s⁻¹) | K_{D} (M) |
|---|---|---|---|
| R6E11 | 3.873E+5 | 2.668E-4 | 6.890E-10 |
| AMG714 | 1.286E+6 | 7.033E-4 | 5.467E-10 |
| CALY-002 | 1.030E+6 | 2.074E-4 | 2.013E-10 |

### Example 5: Humanization of mouse monoclonal antibody R6E11

### 5.1 Humanization of R6E11

Mouse monoclonal antibody R6E11 was humanized to reduce its immunogenicity. A classic framework grafting strategy (J Immunol, 2002 Jul 15; 169(2): 1119-25) ^{[11]} was used for the humanization scheme. The heavy and light chain variable regions of R6E11 were compared to the germline gene sequences of human antibodies in the IMGT database, respectively. Appropriate germline gene sequences were selected to provide the framework regions 1 to 3 of the antibodies (FR1+FR2+FR3), and the appropriate J region genetic sequence was selected to provide the framework region 4 (FR4). This template may be selected based on a variety of factors, such as relative total length of the antibody, CDR size, amino acid residues at the junction between the antibody framework regions (FRs) and the hypervariable regions (CDRs), overall homology of sequences, and the like. The selected template may be a mixture of multiple sequences or may be a consensus template, with the aim of maintaining the appropriate conformation of complementarity determining regions (CDRs) of the parent as much as possible. Finally, one humanized heavy chain variable region mutant, R6E11 VH-h3, and one humanized light chain variable region mutant, R6E11VK-h3, were obtained.

### 5.2 Screening of humanized R6E11 mutants

Given that the heavy chain sequence of the humanized mutant R6E11VH-h3+R6E11VK-h3 included the deamino sites NG and NA, R6E11VH-h3 was designed for mutagenesis to improve its physicochemical properties. The mutagenesis scheme is shown in Table 5. The constructed library had a capacity of 2E+7 and an accuracy of 47%.

**Table 5. Mutagenesis scheme based on library of R6E11VH-h3 mutants**

| Original amino acid (Kabat numbering) | Designed amino acid | Degenerate codon |
|---|---|---|
| 60N | N, D, S, orG | RRC |
| 61A | A, T, or P | VCA |
| 62A | A or S | KCA |
| 63F | F or L | YTC |
| 64M | M or K | AWG |
| 95N | N, D, G, or S | RRC |
| 96G | G, S, A, or T | RSC |

Based on the recombinant library phage display system (see Example 1 in Chinese Patent Application No. 202210871809.6), the prepared library of R6E11VH-h3 mutants and the fully human light chain antibody library were recombined to construct a library of recombinant Fab mutants. The constructed library of recombinant Fab mutants was subjected to three rounds of screening and enrichment by solid phase screening using the human IL-15+IL-15Rα superagonist as an antigen. Finally, a monoclonal antibody, R26H10 (the amino acid sequence of its heavy chain variable region R26H10VH is set forth in SEQ ID NO: 22; and the amino acid sequence of its light chain variable region R26H10VK is set forth in SEQ ID NO: 23), with improved physicochemical properties and containing the fully human light chain, was obtained.

### 5.3 Affinity analysis of humanized R6E11 mutant

Referring to Example 2.2, the affinity of anti-human IL-15 mouse monoclonal antibody R6E11 and its humanized mutant was analyzed with Biacore T200. Results are shown in Table 6.

**Table 6. Affinity constants for binding of anti-human IL-15 mouse monoclonal antibody R6E11 and its humanized mutant to human IL-15 (IL-15+IL-15Rα complex)**

| | Kₐ (M⁻¹s⁻¹) | K_{d} (s⁻¹) | K_{D} (M) |
|---|---|---|---|
| R6E11 | 4.059E+5 | 3.989E-4 | 9.828E-10 |
| R26H10 | 2.522E+5 | 3.077E-4 | 1.220E-9 |

### Example 6: Binding and affinity analysis of anti-human IL-15 monoclonal antibody to IL-15 from different species

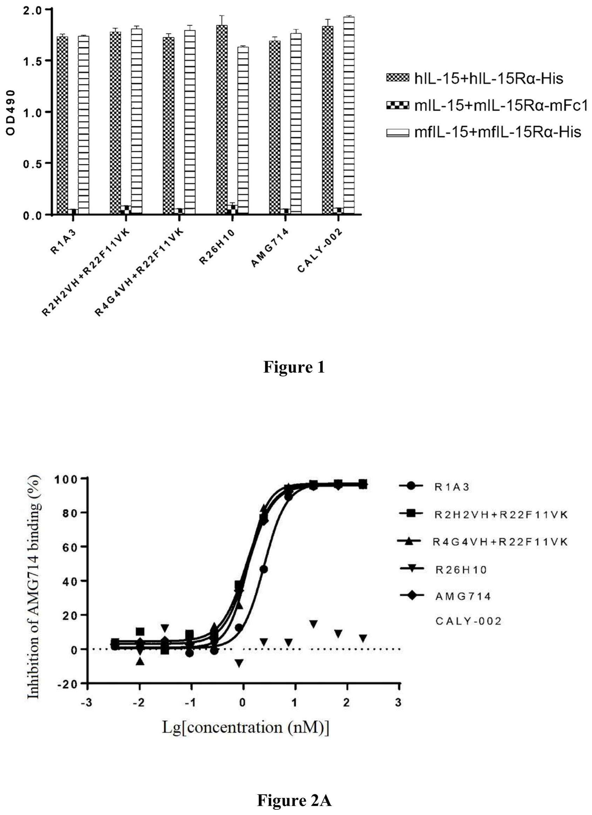

Prepared human IL-15 (hIL-15+hIL-15Rα-His complex), cynomolgus monkey IL-15 (mfIL-15+mfIL-15Rα-His complex), and mouse IL-15 (mIL-15+mIL-15Rα-mFe1 complex) were each coated on 96-well ELISA plates at 1µg/mL, 100µL/well, and overnight at 4°C. After blocking with a blocking solution (3% skim milk-PBST) at 37°C for 1 hour, each anti-IL-15 monoclonal antibody was added separately and bound at 37°C for 1 hour. The ELISA plates were washed with PBST, and added with HRP mouse anti-human IgG (Beijing Biosynthesis Biotechnology Co., Ltd., bsm-0297M-HRP) for binding at 37°C for 1 hour. The ELISA plates were washed with PBST, and added with a solution of chromogenic OPD substrate. After 5-10 minutes, the development was terminated with 1M of H₂SO₄, and optical density values were measured with a microplate reader at dual wavelengths of 492nm/630nm. Results of the ELISA analysis (Figure 1) show that anti-human IL-15 antibodies R1A3, R2H2VH+R22F11VK, R4G4VH+R22F11VK, and R26H10 all cross-recognized human IL-15 and cynomolgus monkey IL-15, while all of the molecules did not recognize mouse IL-15.

Referring to Example 2.2, the affinity of anti-human IL-15 monoclonal antibodies binding to human IL-15 and cynomolgus monkey IL-15 was analyzed with Biacore T200. Results are shown in Table 7 and Table 8.

**Table 7. Affinity constants for binding of anti-human IL-15 monoclonal antibodies to human IL-15 (hIL-15+hIL-15Rα complex)**

| | Kₐ (M⁻¹s⁻¹) | K_{d} (s⁻¹) | K_{D} (M) |
|---|---|---|---|
| R1A3 | 6.209E+5 | 3.801E-4 | 6.122E-10 |
| R2H2VH+R22F11VK | 3.570E+5 | 2.036E-4 | 5.703E-10 |
| R4G4VH+R22F11VK | 4.495E+5 | 1.956E-4 | 4.351E-10 |
| R26H10 | 2.083E+5 | 2.358E-4 | 1.132E-9 |
| AMG714 | 5.123E+5 | 7.616E-4 | 1.487E-9 |
| CALY-002 | 6.969E+5 | 2.352E-4 | 3.374E-10 |

**Table 8. Affinity constants for binding of anti-human IL-15 monoclonal antibodies to cynomolgus monkey IL-15 (mfIL-15+mfIL-15Rα complex)**

| | Kₐ (M⁻¹s⁻¹) | K_{d} (s⁻¹) | K_{D} (M) |
|---|---|---|---|
| R1A3 | 5.519E+5 | 3.983E-4 | 7.216E-10 |
| R2H2VH+R22F11VK | 3.650E+5 | 1.712E-4 | 4.629E-10 |
| R4G4VH+R22F11VK | 3.844E+5 | 1.723E-4 | 4.482E-10 |
| R26H10 | 2.104E+5 | 2.102E-2 | 9.991E-8 |
| AMG714 | 5.834E+5 | 1.237E-3 | 2.120E-9 |
| CALY-002 | 1.274E+6 | 1.882E-4 | 1.477E-10 |

### Example 7: Epitope analysis of anti-human IL-15 monoclonal antibodies

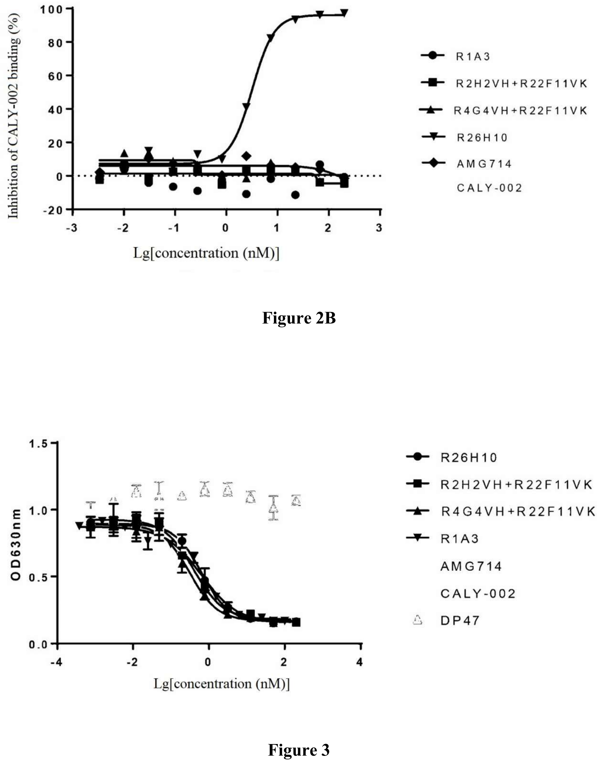

Human IL-15 (hIL-15+hIL-15Rα-his complex) was coated on 96-well ELISA plates at 1µg/mL, 100µL/well, and overnight at 4°C. The blocking solution (3% skim milk-PBST) was used to block at 37°C for 1 hour. Serial dilutions of the anti-human IL-15 monoclonal antibodies (R1A3, R2H2VH+R22FVK, R4G4VH+R22FVK, R26H, AMG714, and CALY-002) was performed with a fixed concentration of purified anti-human IL-15 phages (AMG714 at 4×10¹¹ cfu/mL, CALY-002 at 1×10¹⁰ cfu/mL). The initial concentration was 30µg/mL, with 3-fold gradient dilutions to obtain a total of 11 concentration gradients. 100µL of the antibody solutions per well were added to the blocked 96-well ELISA plates and incubated at 37°C for 1 hour. The ELISA plates were washed with PBST followed by the addition of a secondary HRP-labeled anti-M13 antibody (Beijing Sino Biological Co., Ltd., 11973-MM05T-H), and incubated at 37°C for 1 hour. The ELISA plates were washed with PBST, and added with the solution of chromogenic OPD substrate. After 5-10 minutes, the development was terminated with 1M H₂SO₄, and optical density values were measured with a microplate reader at dual wavelengths of 492nm/630nm. Results of the ELISA analysis are shown in Figure 2. R26H10 binds to an epitope on human IL-15 that is different from that of AMG714 (Figure 2A), and is similar to or overlaps with that of CALY-002 (Figure 2B). R1A3, R2H2VH+R22F11VK and R4G4VH+R22F11VK bind to epitopes on human IL-15 that are similar to or overlap with that of AMG714 (Figure 2A), and are different from that of CALY-002 (Figure 2B). AMG714 binds to an epitope on human IL-15 that is different from that of CALY-002 (Figure 2B).

### Example 8: Evaluation of activities of anti-human IL-15 monoclonal antibodies on HEK-Blue IL-2 cells

HEK-Blue IL2 cells are HEK293-derived cells stably transfected with human CD25 (IL-2Rα), CD122 (IL-2Rβ), and CD132 (IL-2Rγ) genes, achieving IL-2 signaling through the JAK-STAT5 pathway. Additionally, a STAT5-induced secreted embryonic alkaline phosphatase (SEAP) reporter gene was introduced into the HEK-blue cells. Under stimulation with IL-2 or IL-15, the HEK-Blue IL-2 cells can activate JAK/STAT5 and secrete SEAP. The amount of SEAP can be monitored by a SEAP detection kit, QUANTI-Blue^{™} Solution, thereby enabling the monitoring of IL-2 or IL-15 activity.

The HEK-Blue IL-2 cell line was purchased from Invivogen. DMEM+5% inactivated FBS was prepared as a test medium, and used to resuspend the HEK-Blue IL-2 cells to 5 × 10⁵ cells/mL. Antibodies were diluted with 5ng/mL of human IL-15 (hIL-15+hIL-15Rα complex) as the diluent. The antibody concentration gradient started at 400nM, with 4-fold gradient dilutions to obtain 10 concentration points. 100µL of antibodies and 100µL of cells were mixed and incubated in a carbon dioxide incubator (at 37°C, 5% CO₂) for 20 hours. 20µL of supernatant was collected by centrifugation, mixed with 180µL Quanti-blue (Invivogen, Cat. Code: rep-qbs), and incubated at 37°C for 1-2 hours. Absorbance was measured with a microplate reader (Biotek, model 800TS) at 630nm. Raw data were statistically processed with Graphpad Prism 7.0 software. DP47 antibody (negative control, prepared with reference to U.S. Patent Application US 20160200833A1 ^{[12]}, wherein the amino acid sequences of its heavy chain variable region and light chain variable region are set forth in SEQ ID NOs: 50 and 51, respectively). Results are shown in Figure 3. The activities of four molecules, R1A3, R4G4VH+R22F11VK, R2H2VH+R22F11VK, and R26H10, were higher than those of CALY-002 and AMG-714. Specific IC₅₀ data are shown in Table 9.

**Table 9. Activities of anti-human IL-15 monoclonal antibodies on HEK-Blue IL-2 cells**

| | IC₅₀(nM) |
|---|---|
| R26H10 | 0.646 |
| R4G4VH+R22F11VK | 0.312 |
| R2H2VH+R22F11VK | 0.397 |
| R1A3 | 0.634 |
| AMG-714 | 3.530 |
| CALY-002 | 3.227 |

### Example 9: Evaluation of activities of anti-human IL-15 monoclonal antibodies on NK92 cells

The NK92 cell line, purchased from Nanjing Cobioer Biosciences Co., Ltd., is an IL-2-dependent cell line that under IL-2 starvation, its cell proliferation can be promoted by IL-15. The NK92 cells were starved for one day in advance (in a blank culture without IL-2). On day 2, the cells were resuspended to 5 × 10⁵ cells/mL in a medium without IL-2. Antibodies were diluted with 12ng/mL of human IL-15 (hIL-15+hIL-15Rα-his complex) as the diluent. The antibody concentration gradient started at 400nM, with 4-fold gradient dilutions to obtain 10 concentration points. 100µL of the antibodies and 100µL of the cells were mixed and cultured in a carbon dioxide incubator (at 37°C, 5% CO₂) for 2 days. Cell proliferation was measured with the Cell Titer-Glo^{®} Luminescent Cell Viability Assay (Promega, Catalog number: G7571). Fluorescence was measured across the full wavelength with a microplate reader (Molecular Devices, model SpectraMax I3X). Raw data were statistically processed with Graphpad Prism7.0 software. DP47 antibody was used as the negative control.

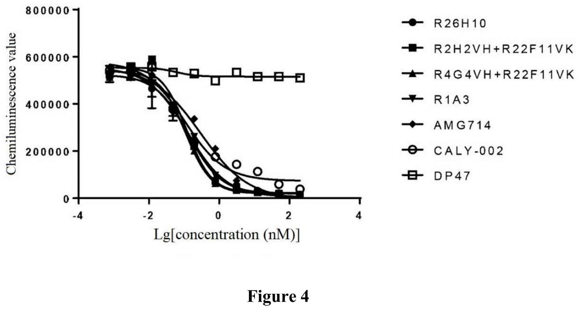

Results are shown in Figure 4. The activities of four anti-human IL-15 monoclonal antibodies, R1A3, R26H10, R4G4VH+R22F11VK, and R2H2VH+R22F11VK, were higher than that of AMG-714, and were similar to that of CALY-002. Among them, CALY-002 did not completely inhibit the activity of IL-15 in the NK92 cells. Specific IC₅₀ data are shown in Table 10.

**Table 10. Activities of anti-human IL-15 monoclonal antibodies on NK92 cells**

| | IC₅₀(nM) |
|---|---|
| R26H10 | 0.133 |
| R4G4VH+R22F11VK | 0.109 |
| R2H2VH+R22F11VK | 0.119 |
| R1A3 | 0.126 |
| AMG-714 | 0.299 |
| CALY-002 | 0.106 |

### Sequence information

**SEQ ID NO: 1** 
NWVNVISDLKKIEDLIQSMHIDATLYTESDVHPSCKVTAMKCFLLELQVISLESGDASIHDTVENLIILANN
SLSSNGNVTESGCKECEELEEKNIKEFLQSFVHIVQMFINTS
**SEQ ID NO: 2** 
ITCPPPMSVEHADIWVKSYSLYSRERYICNSGFKRKAGTSSLTECVLNKATNVAHWTTPSLKCIRDPALVH
QRPAPPSTVTTAGVTPQPESLSPSGKE
**SEQ ID NO: 3** 
NWVNVISDLKKIEDLIQSMHIDATLYTESDVHPSCKVTAMKCFLLELQVISHESGDTDIHDTVENLIILANN
ILSSNGNITESGCKECEELEEKNIKEFLQSFVHIVQMFINTS
**SEQ ID NO: 4** 
ITCPPPVSVEHADIRVKSYSLYSRERYICNSGFKRKAGTSSLTECVLNKATNIAHWTTPSLKCIRDPLLARQ
RPAPPFTVTTAGVTPQPESLSPSGKE
**SEQ ID NO: 5** 
NWIDVRYDLEKIESLIQSIHIDTTLYTDSDFHPSCKVTAMNCFLLELQVILHEYSNMTLNETVRNVLYLANS
TLSSNKNVAESGCKECEELEEKTFTEFLQSFIRIVQMFINTS
**SEQ ID NO: 6** 
GTTCPPPVSIEHADIRVKNYSVNSRERYVCNSGFKRKAGTSTLIECVINKNTNVAHWTTPSLKCIRDPSLA
HYSPVPTVVTPKVTSQPESPSPSAKE
**SEQ ID NO:** 7
   HHHHHH
**SEQ ID NO: 8** 
ASVPRDSGCKPCICTVPEVSSVFIFPPKPKDVLTITLTPKVTCVVVDISKDDPEVQFSWFVDDVEVHTAQTQ
PREEQFNSTFRSVSELPIMHQDWLNGKEFKCRVNSAAFPAPIEKTISKTKGRPKAPQVYTIPPPKEQMAKD
KVSLTCMITDFFPEDITVEWQWNGQPAENYKNTQPIMDTDGSYFVYSKLNVQKSNWEAGNTFTCSVLHE
GLHNHHTEKSLSHSPGK
**SEQ ID NO: 9** 
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPGK
**SEQ ID NO: 10** 
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPEFEGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPASIEKTISKAKGQPREPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPGK
**SEQ ID NO: 11**
**SEQ ID NO: 12** 
GQPKAAPSVTLFPPSSEELQANKATLVCLISDFYPGAVTVAWKADSSPVKAGVETTTPSKQSNNKYAASSY
LSLTPEQWKSHRSYSCQVTHEGSTVEKTVAPTECS
**SEQ ID NO: 13** 
EVQLVQSGAEVKKPGESLKISCKVSGYFFTTYWIGWVRQMPGKGLEYMGIIYPGDSDTRYSPSFQGQVTI
SADKSISTAYLQWSSLKASDTAMYYCARGGHWNAFDFWGQGTLVTVSS
**SEQ ID NO: 14** 
EIVLTQSPGTLSLSPGERATLSCRASQSVSSSYLAWYQQKPGQAPRLLIYGASRRATGIPDRFSGSGSGTDF
TLTISRLEPEDFAVYYCQQFDASQTFGQGTKLEIS
**SEQ ID NO: 15** 
EVQLVQSGAEVKKPGESLKISCKVSGYFFTTYWIGWVRQMPGKGLEYMGIIYPGDSDTRYSPSFQGQVTI
SADKSISTAYLQWSSLKASDTAMYYCARGGNWNCFDYWGQGTLVTVSS
**SEQ ID NO: 16** 
EIVLTQSPGTLSLSPGERATLSCRASQSVSSSYLAWYQQKPGQAPRLLIYGASRRATGIPDRFSGSGSGTDF
TLTISRLEPEDFAVYYCQRYGSSHTFGQGTKLEIS
**SEQ ID NO: 17** 
EVQLVESGGGLVQPGGSLRLSCAASEFTFSNYAMSWVRQAPGKGLEWVATISRGGDYTYYPDSVKGRFTI
SRDNSKNTLYLQMNSLRAEDTAVYYCARRVSMITGGWAMDYWGQGTLVTVSS
**SEQ ID NO: 18** 
DVVMTQSPLSLPVTLGQPASISCRSSQSIVDITGNTYLEWYQQRPGQSPRLLIYKVFNRFSGVPDRFSGSGS
GTDFTLKISRVEAEDVGVYYCFQDSFVPYTFGQGTKLEIK
**SEQ ID NO: 19** 
EVQLVQSGAEVKKPGESLKISCKVSGYFFTTYWIGWVRQMPGKGLEYMGIIYPGDSDTRYSPSFQGQVTI
SADKSISTAYLQWSSLKASDTAMYYCARGGHWNAFDIWGQGTLVTVSS
**SEQ ID NO: 20** 
EIVLTQSPGTLSLSPGERATLSCRASQSVIGSYLAWYQQKPGQAPRLLIYSASKLASGIPDRFSGSGSGTDFT
LTISRLEPEDFAVYYCQQHYSTPGTFGQGTKVEIK
**SEQ ID NO: 21** 
EVQLVQSGAEVKKPGESLKISCKVSGYFFTTYWIGWVRQMPGKGLEYMGIIYPGDSDTRYSPSFQGQVTI
SADKSISTAYLQWSSLKASDTAMYYCARGGHWNSFDTWGQGTLVTVSS
**SEQ ID NO: 22** 
QVTLKESGPVLVKPTETLTLTCTVSGFSLTTYAVHWIRQPPGKALEWLGVIWGAGSTDYNPSFMSRLTISK
DTSKSQVVLTMTNMDPVDTATYYCAKNAAYYVMDYWGQGTTVTVSS
**SEQ ID NO: 23** 
EIVLTQSPGTLSLSPGERATLSCRASQSVIYSYLAWYQQKPGQAPRLLIYAASKLASGIPDRFSGSGSGTDFT
LTISRLEPEDFAVYYCQQRASYPLTFGQGTKVEIK
**SEQ ID NO: 24**
   TGCATTTGAACTCCTTGCC
**SEQ ID NO: 25**
   CCATCAATCTTCCACTTGAC
**SEQ ID NO: 26**
   TYWIG
**SEQ ID NO: 27**
   IIYPGDSDTRYSPSFQG
**SEQ ID NO: 28**
   GGHWNAFDF
**SEQ ID NO: 29**
   GGHWNAFDI
**SEQ ID NO: 30**
   GGHWNSFDT
**SEQ ID NO: 31**
   TYAVH
**SEQ ID NO: 32**
   VIWGAGSTDYNPSFMS
**SEQ ID NO: 33**
   NAAYYVMDY
**SEQ ID NO: 34**
   RASQSVSSSYLA
**SEQ ID NO: 35**
   GASRRAT
**SEQ ID NO: 36**
   QQFDASQT
**SEQ ID NO: 37**
   RASQSVIGSYLA
**SEQ ID NO: 38**
   SASKLAS
**SEQ ID NO: 39**
   QQHYSTPGT
**SEQ ID NO: 40**
   RASQSVIYSYLA
**SEQ ID NO: 41**
   AASKLAS
**SEQ ID NO: 42**
   QQRASYPLT
**SEQ ID NO: 43** 
EVKLKESGPSLVQPSQSLSITCTVSGFSLTTYAVHWVRQSPGKGLEWLGVIWGAGSTDYNAAFMSRLSIT
KDNSKSQVFFKMNSLQADDTAIYFCAKNGAYYVMDYWGQGTSVTVSS
**SEQ ID NO: 44** 
EIVLTQSPAIMSASPGEKVTITCSASSSVSYMHWFQQRPGTSPKLWIYSTSNLASGVPARFSGSGSGTSYSL
TISRMEAEDAATYYCQQRSSYPLTFGAGTKLEIK
**SEQ ID NO: 45**
   VIWGAGSTDYNAAFMS
**SEQ ID NO: 46**
   NGAYYVMDY
**SEQ ID NO: 47**
   SASSSVSYMH
**SEQ ID NO: 48**
   STSNLAS
**SEQ ID NO: 49**
   QQRSSYPLT
**SEQ ID NO: 50** 
EVQLLESGGGLVQPGGSLRLSCAASGFTFSSYAMSWVRQAPGKGLEWVSAISGSGGSTYYADSVKGRFTI
SRDNSKNTLYLQMNSLRAEDTAVYYCAKGSGFDYWGQGTLVTVSS
**SEQ ID NO: 51**

EIVLTQSPGTLSLSPGERATLSCRASQSVSSSYLAWYQQKPGQAPRLLIYGASSRATGIPDRFSGSGSGTDFT
LTISRLEPEDFAVYYCQQYGSSPLTFGQGTKVEIK

### References

1. Steel JC, Waldmann TA, Morris JC. Interleukin-15 biology and its therapeutic implications in cancer. Trends Pharmacol Sci. 2012 Jan;33(1):35-41.
2. Fehniger TA, Caligiuri MA. Interleukin 15: biology and relevance to human disease. Blood. 2001 Jan 1;97(1):14-32.
3. Bergqvist C, Ezzedine K. Vitiligo: A focus on pathogenesis and its therapeutic implications. J Dermatol. 2021 Mar;48(3):252-270.
4. Richmond JM, Strassner JP, Rashighi M, et al. Resident Memory and Recirculating Memory T Cells Cooperate to Maintain Disease in a Mouse Model of Vitiligo. J Invest Dermatol. 2019 Apr;139(4):769-778.
5. Richmond JM, Strassner JP, Zapata L Jr, et al., Antibody blockade of IL-15 signaling has the potential to durably reverse vitiligo. Sci Transl Med. 2018 Jul 18;10(450).
6. Phage Display: A Practical Approach, Clackson, T. (USA) and Lowman, H. B. (USA) (ed.), translated by Lan Ma et al., Chemical Industry Press, May 2008.5.
7. CN202210871809.6.
8. US7153507B2.
9. US10301384B2.
10. Krebber A, Bornhauser S, Burmester J, et al. Reliable cloning of functional antibody variable domains from hybridomas and spleen cell repertoires employing a reengineered phage display system. J Immunol Methods. 1997;201(1):35-55.
11. J Immunol, 2002 Jul 15; 169(2): 1119-25.

## Claims

1. An antibody that binds to human IL-15, comprising a heavy chain variable region comprising amino acid sequences of HCDR1, HCDR2, and HCDR3, and a light chain variable region comprising amino acid sequences of LCDR1, LCDR2, and LCDR3, wherein
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 29, the LCDR1 has the amino acid sequence set forth in SEQ ID NO: 37, the LCDR2 has the amino acid sequence set forth in SEQ ID NO: 38, and the LCDR3 has the amino acid sequence set forth in SEQ ID NO: 39;
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 28, the LCDR1 has the amino acid sequence set forth in SEQ ID NO: 34, the LCDR2 has the amino acid sequence set forth in SEQ ID NO: 35, and the LCDR3 has the amino acid sequence set forth in SEQ ID NO: 36;
the HCDR1 has the amino acid sequence set forth in SEQ ID NO: 26, the HCDR2 has the amino acid sequence set forth in SEQ ID NO: 27, the HCDR3 has the amino acid sequence set forth in SEQ ID NO: 30, the LCDR1 has the amino acid sequence set forth in SEQ ID NO: 37, the LCDR2 has the amino acid sequence set forth in SEQ ID NO: 38, and the LCDR3 has the amino acid sequence set forth in SEQ ID NO: 39;
the HCDR1 has the amino acid sequence set forth in SEQ ID NO: 31, the HCDR2 has the amino acid sequence set forth in SEQ ID NO: 32, the HCDR3 has the amino acid sequence set forth in SEQ ID NO: 33, the LCDR1 has the amino acid sequence set forth in SEQ ID NO: 40, the LCDR2 has the amino acid sequence set forth in SEQ ID NO: 41, and the LCDR3 has the amino acid sequence set forth in SEQ ID NO: 42; or
the HCDR1 has the amino acid sequence set forth in SEQ ID NO: 31, the HCDR2 has the amino acid sequence set forth in SEQ ID NO: 45, the HCDR3 has the amino acid sequence set forth in SEQ ID NO: 46, the LCDR1 has the amino acid sequence set forth in SEQ ID NO: 47, the LCDR2 has the amino acid sequence set forth in SEQ ID NO: 48, and the LCDR3 has the amino acid sequence set forth in SEQ ID NO: 49; and
wherein the amino acid sequences of the HCDRs and the LCDRs are defined according to Kabat.

2. The antibody of claim 1, wherein the antibody comprises a heavy chain variable region having the amino acid sequence set forth in SEQ ID NO: 13, 19, 21, 22 or 43.

3. The antibody of claim 1, wherein the antibody comprises a light chain variable region having the amino acid sequence set forth in SEQ ID NO: 14, 20, 23 or 44.

4. The antibody of any one of claims 1 to 3, wherein
the antibody comprises a heavy chain variable region having the amino acid sequence set forth in SEQ ID NO: 13, and a light chain variable region having the amino acid sequence set forth in SEQ ID NO: 14;
the antibody comprises a heavy chain variable region having the amino acid sequence set forth in SEQ ID NO: 19, and a light chain variable region having the amino acid sequence is set forth in SEQ ID NO: 20;
the antibody comprises a heavy chain variable region having the amino acid sequence set forth in SEQ ID NO: 21, and a light chain variable region having the amino acid sequence set forth in SEQ ID NO: 20;
the antibody comprises a heavy chain variable region having the amino acid sequence set forth in SEQ ID NO: 22, and a light chain variable region having the amino acid sequence set forth in SEQ ID NO: 23; or
the antibody comprises a heavy chain variable region having the amino acid sequence set forth in SEQ ID NO: 43, and a light chain variable region having the amino acid sequence set forth in SEQ ID NO: 44.

5. An antibody that binds to human IL-15, wherein the antibody comprises a heavy chain variable region having an amino acid sequence that has at least 90% identity to any one of SEQ ID NOs: 13, 19, 21, 22 and 43, and a light chain variable region having an amino acid sequence that has at least 90% identity to any one of SEQ ID NOs: 14, 20, 23 and 44.

6. The antibody of any one of claims 1 to 5, wherein
the antibody is a whole antibody, a Fab fragment, an F(ab')₂ fragment, or a single chain Fv fragment (scFv); preferably, the antibody is a fully human antibody or a humanized antibody; and/or
the antibody is a monoclonal antibody; and/or
the antibody further comprises a heavy chain constant region selected from IgG1 subtype, IgG2 subtype, or IgG4 subtype; preferably, the heavy chain constant region is of IgG1 subtype; more preferably, the heavy chain constant region is of IgG1m3 subtype; and/or
a Fc fragment comprises the amino acid F at position 234, the amino acid E at position 235, and the amino acid S at position 331; and/or
the heavy chain constant region comprises the amino acid Y at position 252, the amino acid T at position 254, and the amino acid E at position 256; and/or
the antibody further comprises a light chain constant region selected from kappa subtype or lambda subtype; preferably, the light chain constant region is of kappa subtype; and
wherein amino acid positions of the antibody constant regions are determined according to EU numbering.

7. The antibody of any one of claims 1 to 6, wherein
the antibody binds to human IL-15 and/or monkey IL-15; and/or
the antibody is capable of inhibiting the activity of IL-15, such as inhibiting the ability of IL-15 to induce secretion of secreted embryonic alkaline phosphatase (SEAP) and/or inhibiting the ability of IL-15 to induce proliferation of NK92 cells.

8. A nucleic acid molecule encoding the antibody of any one of claims 1 to 7.

9. A pharmaceutical composition comprising the antibody of any one of claims 1 to 7, and a pharmaceutically acceptable excipient, diluent, or carrier.

10. The pharmaceutical composition of claim 9, for use in the prevention or treatment of an IL-15-mediated disease; preferably, wherein the IL-15-mediated disease is selected from the group consisting of vitiligo, celiac disease, eosinophilic esophagitis, and large granular lymphocytic leukemia.

11. Use of the antibody of any one of claims 1 to 7, or the pharmaceutical composition of claim 9 or 10 in the manufacture of a medicament for the prevention or treatment of an IL-15-mediated disease; preferably, wherein the IL-15-mediated disease is selected from the group consisting of vitiligo, celiac disease, eosinophilic esophagitis, and large granular lymphocytic leukemia.

12. A method of preventing or treating an IL-15-mediated disease comprising administering to a subject in need thereof the antibody of any one of claims 1 to 7, or the pharmaceutical composition of claim 9 or 10; preferably, wherein the IL-15-mediated disease is selected from the group consisting of vitiligo, celiac disease, eosinophilic esophagitis, and large granular lymphocytic leukemia.
